# EUROPEAN PATENT APPLICATION

(11) **EP 3 892 193 A1**
(43) Date of publication of application: **13.10.2021**
(21) Application number: 21167694.5
(22) Date of filing: 09.04.2021
(51) Int. Cl.: A61B 5/08, A61B 5/097, A61B 5/087, G01N 33/497

(54) **PNEUMONIA DETECTION DEVICE**

(30) Priority: 09.04.2020 TW 109112037
(71) Applicant: AI Nose Corporation, New Taipei City 24250 (TW)
(72) Inventor: WENG, Tzu-Ting, 35053 MIAOLI COUNTY (TW); HUANG, Chia-Pin, 35053 MIAOLI COUNTY (TW); LIAO, Yu-Hsuan, 35053 MIAOLI COUNTY (TW); TSAI, Chun-Hsien, 35053 MIAOLI COUNTY (TW); LEE, Ting-Chuan, 35053 MIAOLI COUNTY (TW); TSAI, Chun-Jung, 35053 MIAOLI COUNTY (TW)
(74) Representative: 2K Patentanwälte Blasberg Kewitz & Reichel

(57) **Abstract**

The present invention provides a pneumonia detection device for detecting an exhaled air (101) of a patient (100) to determine whether the lungs of the patient (100) have been infected by at least one bacterial species. The device comprises a multi-gas sensing module (10), a temperature and humidity control module (20), and an operation control unit (30). The multi-gas sensing module (10) comprises a gas sensor array (12) reacting with a gas metabolized from the bacteria to generate a plurality of characteristic signals. The temperature and humidity control module (20) controls and measures an operational condition of 45°C-60°C and humidity between 7% and 20% for the gas sensor array (12). The gas sensor array (12) contacts with the exhaled air (101) to generate a plurality of measurement signals. The operation control unit (30) comparisons the measurement signals and the characteristic signals of different bacteria to generate a result determining whether the patient (100) has contracted the bacteria.

## Description

### FIELD OF THE INVENTION

The present invention relates to a medical device, and particularly to a pneumonia detection device.

### BACKGROUND OF THE INVENTION

Ventilator-associated pneumonia (VAP) is the most common cause of death in the intensive care unit (ICU) with an infection rate of about 5∼67% and the infection fatality rate to be as high as 20-50% that prolongs the inpatient days for 4-9 days and causes heavy burdens for hospitals.

Pneumonia is caused by bacterial invasion of the lungs and drug administration of a targeted antibiotic is prescribed depending on the type of bacteria. However, in the early stages of infection, patients usually have pathological states of fever and lung infiltration and clinically can only be diagnosed as suspected pneumonia. In the initial drug administration, only broad-spectrum antibiotics can be used. Patients must undergo procedures such as sputum suctioning, bacterial culture, etc. in order to determine the bacterial species of the infection and administer targeted antibiotics. However, it takes 3-5 days for bacterial culture. Problems might also occur while determining the bacteria, such as poor sampling, culture contamination, etc.

In order to quickly identify the type of bacteria, there are already targeted diagnostic reagents that can be used, such as rapid K1/K2 testing reagent for Klebsiella pneumoniae virulent strains, disclosed in the Taiwan Patent No. 1384075, which determines whether a person is infected with Klebsiella *pneumoniae* virulent strain K1/K2 within 3-5 minutes.

However, the aforementioned technique of the prior art still requires obtaining specimens from the patient for testing. When the bacterial concentration is low, the bacteria still need to be cultured in order to increase the test accuracy. In clinical practice, the use of a single-use rapid test kit still requires the judgment of a doctor, who would only use the kit when the clinical symptoms of a patient show the possible infection of the virulent strains K1/K2 of Klebsiella *pneumoniae,* for the sake of cost saving.

### SUMMARY OF THE INVENTION

The primary objective of the present invention is to solve the problem of the prior art of not being able to rapidly test the patient and determine whether the patient is infected with pneumonia.

To achieve the aforementioned objective, the present invention provides a pneumonia detection device, which detects an exhaled air of a patient to determine whether the lungs of the patient have been infected by at least one bacterial species. The pneumonia detection device comprises a multi-gas sensing module, a temperature and humidity control module, and an operation control unit. The multi-gas sensing module comprises a chamber, a gas sensor array disposed in the chamber to react with a gas metabolized from the at least one bacterial species to generate a plurality of characteristic signals, an air inlet tube guiding the exhaled air entering the chamber, an air inlet valve installed on the air inlet tube to control whether the exhaled air entering the chamber, and an air outlet valve installed on the chamber to control whether the exhaled air discharging from the chamber, wherein the chamber and the air inlet tube are connected to form an air channel. The temperature and humidity control module controls and measures a temperature and a humidity within the air channel. The operation control unit connects to the multi-gas sensing module and the temperature and humidity control module. The temperature and humidity control module controls and adjusts an operational condition of the temperature between 45°C and 60°C and the humidity between 7% and 20% within the chamber during detection, the gas sensor array contacts with the exhaled air to generate a plurality of measurement signals, the operation control unit then generates a result regarding whether the patient has infected the at least one bacterial species according to the plurality of measurement signals and the plurality of characteristic signals of the at least one bacterial species

Therefore, the pneumonia detection device of the present invention comprises a gas sensor array which is able to sample multiple sets of measurement signals, and is able to set the operational condition of the gas sensor array under the temperature between 45°C to 60°C and the humidity between 7% and 20%, to increase the accuracy of the measurement.

### BRIEF DESCRIPTION OF THE DRAWINGS

Fig. 1 is a schematic diagram of the appearance of a pneumonia detection device of one embodiment of the present invention;
Fig. 2 is a block diagram of the pneumonia detection device of one embodiment of the present invention;
Fig. 3 is a schematic diagram of the installation and operation of one embodiment of the present invention;
Fig. 4 is a schematic diagram of the operational process of one embodiment of one embodiment of the present invention; and
Fig. 5 is a receiver operating characteristic (ROC) curve of one experimental example of the present invention.

### DETAILED DESCRIPTION OF THE PREFERRED EMBODIMENTS

Details and technical content, features, and effect of the present invention are given with the accompanying drawings below.

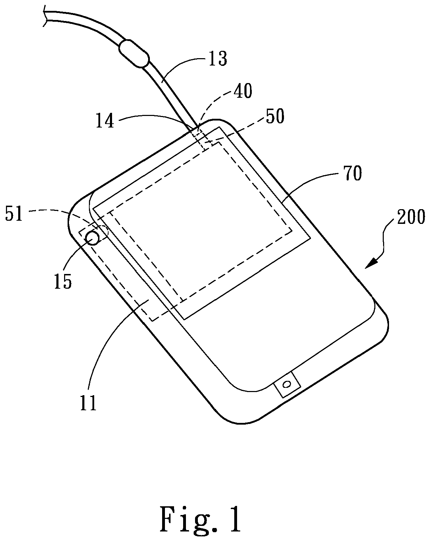

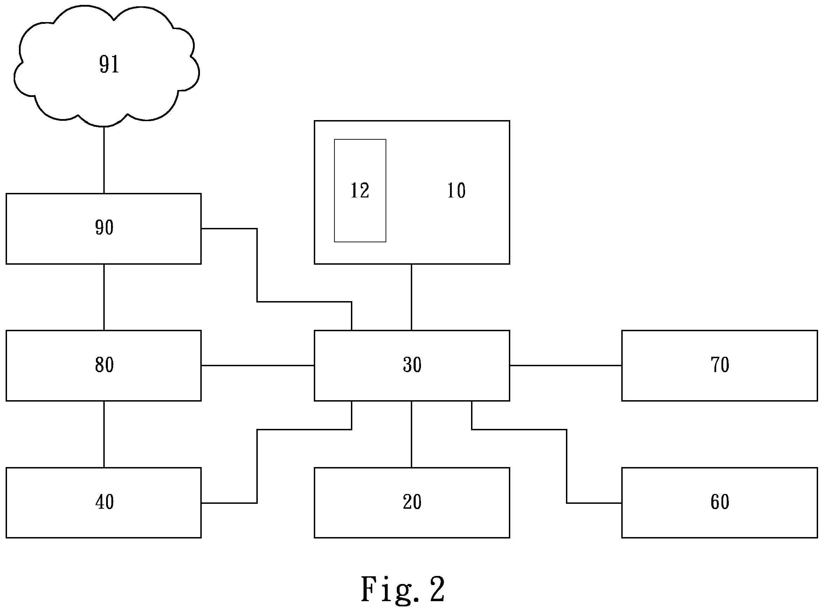

Please refer to Fig. 1, Fig. 2 and Fig. 3. The present invention provides a pneumonia detection device, which detects an exhaled air 101 of a patient 100 to determine whether lungs of the patient 100 have been infected by at least one bacterial species. The pneumonia detection device comprises a multi-gas sensing module 10, a temperature and humidity control module 20, and an operation control unit 30, wherein the multi-gas sensing module 10, the temperature and humidity control module 20, and the operation control unit 30 are installed within a casing 200.

The multi-gas sensing module 10 comprises a chamber 11, a gas sensor array 12, an air inlet tube 13, an air inlet valve 14, and an air outlet valve 15. The gas sensor array 12 is disposed in the chamber 11 to react with a gas metabolized from the at least one bacterial species to generate a plurality of characteristic signals, wherein different bacteria species metabolizes different gases that generate different characteristic signals. In one embodiment, the gas sensor array 12 includes a plurality of sensors, and each of the plurality of sensors includes at least one metallic oxide and one catalytic material. The at least one metallic oxide is selected from the group consisting of Al₂O₃, TiO₂, Fe₂O₃, CeO₂, CuO, ZnO, SiO₂, V₂O₅, MgO, La₂O₃, SnO₂, MnO₂, MoO3, Mo₂O₅ and a combination thereof, and the catalytic material is selected from the group consisting of WO₃, Au, Pt, Pd, Ru, Ir, Os, Co, Fe and a combination thereof. The metallic oxide and the catalytic material are selected according to the actual applications.

The air inlet tube 13 guides the exhaled air 101 entering the chamber 11. The air inlet valve 14 is installed on the air inlet tube 13 to control whether the exhaled air 101 entering the chamber 11. The air outlet valve 15 is installed on the chamber 11 to control whether the exhaled air 101 discharging from the chamber 11. The chamber 11 and the air inlet tube 13 are connected to form an air channel.

The temperature and humidity control module 20 controls and measures the temperature and humidity within the air channel. The operation control unit 30 is connected to the multi-gas sensing module 10 and the temperature and humidity control module 20. In the present invention, the chamber 11 is kept in an operational condition of temperature between 45°C and 60°C and humidity between 7% and 20% during detection. The gas sensor array 12 contacts with the exhaled air 101 to generate a plurality of measurement signals. The operation control unit 30 then generates a result regarding whether the patient 100 has infected the at least one bacterial species according to the plurality of measurement signals and the plurality of characteristic signals of the at least one bacterial species, wherein the result includes whether the patient 100 is infected with the at least one bacterial species and the species type of the at least one bacterial species, etc.

In one embodiment, the pneumonia detection device further comprises a flow sensor 40, a first suction motor 50, a second suction motor 51, an air pressure sensor 60, a display screen 70, an analog-to-digital converter 80, and a database unit 90. The flow sensor 40 is installed on the air inlet tube 13 to measure a flow of the exhaled air 101 passing through the air inlet tube 13. The flow sensor 40 is connected with the operation control unit 30. The first suction motor 50 is installed on the air inlet tube 13 to draw the exhaled air into the chamber 11. The second suction motor 51 is connected to the chamber 11 to discharge air out of the chamber 11. The air pressure sensor 60 is installed within the chamber 11 to monitor the air pressure of the chamber 11. The display screen 70 is connected to the operation control unit 30 to display the result. The analog-to-digital converter 80 is connected to the operation control unit 30. The analog-to-digital converter 80 processes the noise generated by the sensor since the structure thereof is deformed due to the temperature variation, for example, the analog-to-digital converter 80 filters the noise and amplifies the signal. The database unit 90 is connected with the operation control unit 30 and stores the plurality of characteristic signals and the plurality of measurement signals. The database unit 90 is connected with a cloud database 91.

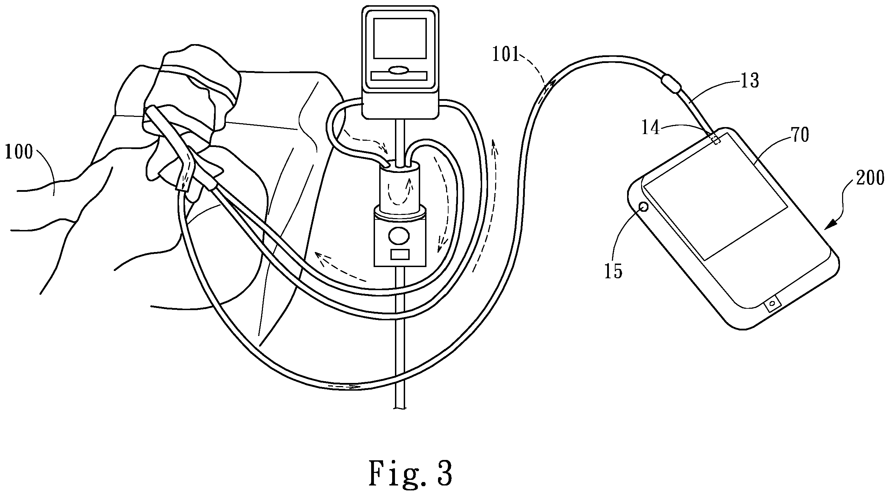

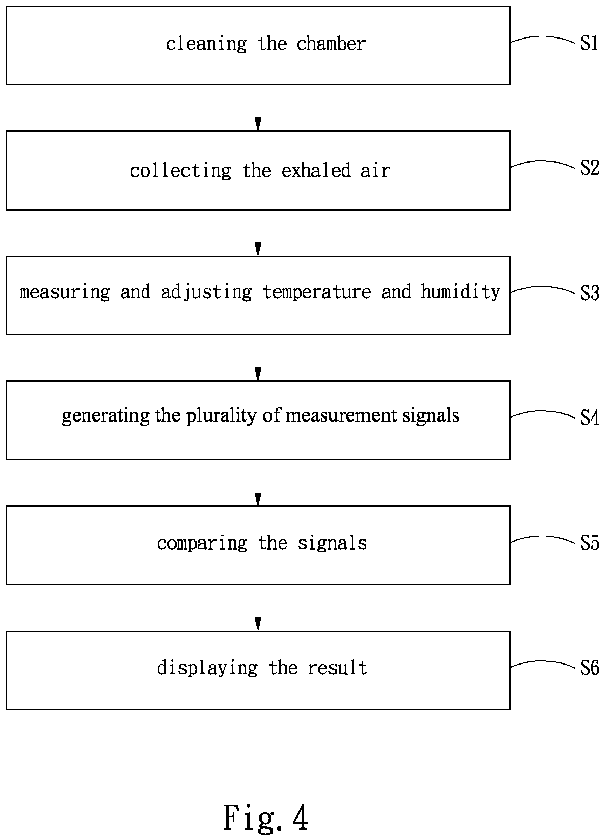

Please refer to Fig. 4. An operational process of the present invention includes step S1: cleaning the chamber, step S2: collecting the exhaled air, step S3: measuring and adjusting temperature and humidity, step S4: generating the plurality of measurement signals, step S5: comparing the signals, and step S6: displaying the result. Descriptions of these steps are provided as follows.
step S1: cleaning the chamber. In order to increase the accuracy of the measurement, opening the air inlet valve 14 and the air outlet valve 15, then turning on the first suction motor 50 and the second suction motor 51 to clean the chamber 11 and the air inlet tube 13 with air or inert gas;
step S2: collecting the exhaled air. As shown in Fig. 3, after cleaning the chamber 11, closing the air inlet valve 14 and keeping the air outlet valve 15 open, then turning on the second suction motor 51 to draw out the air in the chamber 11 in order to maintain a negative pressure within the chamber 11. Next, closing the air outlet valve 15 and turning off the second suction motor, then opening the air inlet valve 14 and turning on the first suction motor 50 to draw the exhaled air 101 into the chamber 11 to generate a positive pressure environment. The flow sensor 40 measures the flow of the exhaled air 101 passing through the air inlet tube 13. The air pressure sensor 60 measures the pressure inside the chamber 11 to ensure sufficient volume of the exhaled air 101 is collected;
step S3: measuring and adjusting temperature and humidity. In an embodiment, the temperature and humidity control module includes a temperature sensing element, a humidity sensing element, a humidifying element, a dehumidifying element, a heating element, and a cooling element, so as to keep the temperature of the chamber 11 between 45n and 60d, and to keep the humidity of the chamber 11 between 7% and 20%, thereby meet the requirements of the subsequent measurements;
step S4: generating the plurality of measurement signals. The gas sensor array 12 contacts with the exhaled air 101 and generates plurality of measurement signals, which correspond to the gas sensor array 12. The gas sensor array 12 comprises a plurality of gas sensor chips arranged in multiple matrices that respectively generate the plurality of measurement signals based on the characteristics of the exhaled air 101;
step S5: comparing the signals. The operation control unit 30 comparisons the plurality of measurement signals and the plurality of characteristic signals of bacteria species. Since different bacteria metabolisms generate different gases, a result that determines whether the patient 100 has contracted the bacteria can be produced based on the outcome of the comparison. The result includes whether a patient is infected with the bacterium and the bacterial species of the bacterium. In practice, the analog-to-digital converter 80 converts analog signals to digital signals for ease of comparison. Meanwhile, the plurality of characteristic signals and the plurality of measurement signals used for comparison can be stored in the database unit 90, and can be uploaded to and downloaded from the cloud database 91; and
step S6: displaying the result. The display screen 70 displays the result so that doctors can prescribe targeted antibiotics based on the bacterial species of the bacterium in order to inhibit the bacterium from reproduction or growth.

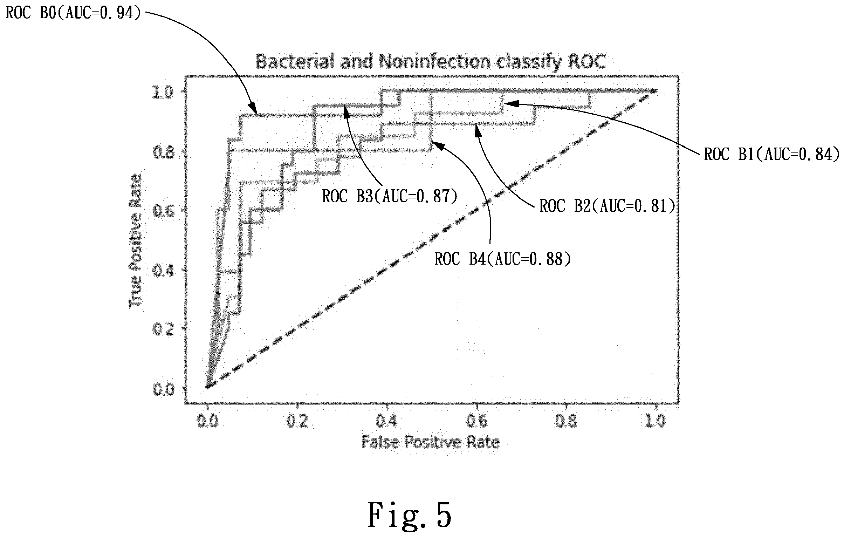

Please refer to Fig. 5, which shows a receiver operating characteristic (ROC) curve of one experimental example of the present invention based on the test results of ventilator-associated pneumonia (VAP) performed on 221 subjects using the pneumonia detection device of the present invention, wherein the ROC B0 curve represents Pseudomonas *aeruginosa* and the area under the curve (AUC) value is 0.94; the ROC B1 curve represents Klebsiella *pneumoniae* and the AUC value is 0.84; the ROC B2 curve represents Acinetobacter *baumannii* and the AUC value is 0.81; the ROC B3 curve represents Escherichia *coli* and the AUC value is 0.87; the ROC B4 curve represents Staphylococcus and the AUC value is 0.88. The results in Fig. 5 shows that tests carried out using the pneumonia detection device of the present invention can accurately detect VAP.

In summary of the aforementioned descriptions, the pneumonia detection device of the present invention comprises a gas sensor array that is able to sample multiple sets of measurement signals, and is able to set the operational condition of the gas sensor array under the temperature between 45°C and 60°Cand the humidity between 7% and 20% , so that the accuracy of the measurement can be increased.

## Claims

1. A pneumonia detection device for detecting an exhaled air (101) of a patient (100) to determine whether lungs of the patient (100) have been infected by at least one bacterial species, comprising:
a multi-gas sensing module (10), comprising a chamber (11), a gas sensor array (12) disposed in the chamber (11) to react with a gas metabolized from the at least one bacterial species to generate a plurality of characteristic signals, an air inlet tube (13) guiding the exhaled air (101) entering the chamber (11), an air inlet valve (14) installed on the air inlet tube (13) to control whether the exhaled air (101) entering the chamber (11), and an air outlet valve (15) installed on the chamber (11) to control whether the exhaled air (101) discharging from the chamber (11), wherein the chamber (11) and the air inlet tube (13) are connected to form an air channel;
a temperature and humidity control module (20), controlling and measuring a temperature and a humidity within the air channel; and
an operation control unit (30), connecting to the multi-gas sensing module (10) and the temperature and humidity control module (20);
wherein the temperature and humidity control module (20) controls and adjusts an operational condition of the temperature between 45°C and 60°C and the humidity between 7% and 20% within the chamber (11) during detection, the gas sensor array (12) contacts with the exhaled air (101) to generate a plurality of measurement signals, the operation control unit (30) then generates a result regarding whether the patient (100) has infected the at least one bacterial species according to the plurality of measurement signals and the plurality of characteristic signals of the at least one bacterial species.

2. A pneumonia detection device according to claim 1, wherein the pneumonia detection device further comprises a flow sensor (40) installed on the air inlet tube (13) to measure a flow of the exhaled air (101) passing through the air inlet tube (13), and the flow sensor (40) is connected with the operation control unit (30).

3. A pneumonia detection device according to claim 1, wherein the pneumonia detection device further comprises a first suction motor (50) installed on the air inlet tube (13) to draw the exhaled air (101) into the chamber (11).

4. A pneumonia detection device according to claim 1 or 3, wherein the pneumonia detection device further comprises a second suction motor (51) connected to the chamber (11) to discharge air out of the chamber (11).

5. A pneumonia detection device according to claim 4, wherein the pneumonia detection device further comprises an air pressure sensor (60) installed within the chamber (11).

6. A pneumonia detection device according to claim 1, wherein the pneumonia detection device further comprises a display screen (70) connected to the operation control unit (30) to display the result.

7. A pneumonia detection device according to claim 1, wherein the pneumonia detection device further comprises an analog-to-digital converter (80) connected to the operation control unit (30).

8. A pneumonia detection device according to claim 1, wherein the pneumonia detection device further comprises a database unit (90) connected with the operation control unit (30) to store the plurality of characteristic signals and the plurality of measurement signals.

9. A pneumonia detection device according to claim 8, wherein the database unit (90) is connected with a cloud database (91).
